(19) Europäisches Patentamt
European Patent Office
Office européen des brevets

(11) **EP 2 926 739 B1**

(12) **EUROPEAN PATENT SPECIFICATION**

(45) Date of publication and mention
of the grant of the patent:
**18.10.2017 Bulletin 2017/42**

(51) Int Cl.:
***A61B 8/08*** (2006.01)

(21) Application number: **15161294.2**

(22) Date of filing: **27.03.2015**

(54) **ACQUISITION CONTROL FOR ELASTICITY ULTRASOUND IMAGING**

ERFASSUNGSSTEUERUNG FÜR ELASTIZITÄTSULTRASCHALLBILDGEBUNG

COMMANDE D'ACQUISITION POUR UNE IMAGERIE PAR ULTRASONS D'ÉLASTICITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2014 US 201414231425**

(43) Date of publication of application:
**07.10.2015 Bulletin 2015/41**

(73) Proprietor: **Siemens Medical Solutions USA, Inc.
Malvern, PA 19355-1406 (US)**

(72) Inventors:
• **Freiburger, Paul D.
Seattle, WA Washington 98112 (US)**
• **Ivancevich, Nikolas M.
Seattle, WA Washington 98117 (US)**
• **Fan, Liexiang
Sammamish, WA Washington 98075 (US)**

(74) Representative: **Patentanwälte Bals & Vogel
Sendlinger Strasse 42A
80331 München (DE)**

(56) References cited:
**EP-A1- 1 782 736       US-A1- 2012 092 527
US-A1- 2013 123 630**

**Description**

BACKGROUND

The present embodiments relate to elasticity ultrasound imaging. Elasticity imaging includes acoustic radiation force impulse (ARFI) imaging, shear wave imaging, strain imaging, and/or strain rate imaging. Elasticity is a type of parametric imaging measuring tissue characteristics. Tissue with different characteristics responds to stress differently. The tissue response to stress is measured. For example, by transmitting an ARFI pushing pulse, ultrasound may be used to displace tissue directly or through generation of a shear or longitudinal wave. The displacement resulting from the pushing pulse may be measured using further ultrasound scanning. Where the displacement response to the longitudinal wave is measured, ARFI imaging is provided. Where the displacement response to the shear wave is measured, shear imaging is provided.

Pushing pulses are repeated to measure displacement at different laterally spaced locations. However, the repetition of ARFI pulses may cause undesired transducer heating and introduce delays in scanning. Elasticity imaging, such as ARFI or shear wave elastography imaging, typically requires a longer time to acquire data than traditional ultrasound imaging modes, like B-mode or Spectral Doppler (e.g., several milliseconds versus hundreds of microseconds). This longer acquisition time may be an issue if a sonographer is trying to search to locate a target or if the target itself has too much movement. A sonographer typically estimates a compromise between motion compensation, frame rate, resolution, and image quality as part of configuring the system for acquiring elasticity images. This use of predetermined settings fixed for the elasticity imaging does not deal with changes in motion and may not be optimal for existing motion.

The prior art application EP 1 782 736 A1 discloses a system and a corresponding method for ultrasonic elasticity imaging in which a first and a second acquisition mode can be employed. The acquisition modes can differ, e.g. in the frame rate or in the resolution.

BRIEF SUMMARY

By way of introduction, the preferred embodiments described below include methods, instructions, and systems for acquisition control for elasticity imaging. Acquisition settings for elasticity imaging are controlled based on detected motion. Rather than rely solely on user settings, one or more acquisition parameters are automatically set based on the detected motion. For example, the settings provide for a more rapid frame rate with less resolution in elasticity imaging for times associated with greater motion and for less rapid frame rate with greater resolution for times associated with lesser motion.

In a first aspect, a method is provided for acquisition control for elasticity ultrasound imaging. Motion of an ultrasound transducer, a patient being scanned by the ultrasound transducer, or both is detected. A processor calculates a value of an acquisition parameter. The value is calculated to provide a frame rate based on an amount of the detected motion. Elasticity ultrasound imaging uses the value of the acquisition parameter so that the elasticity ultrasound imaging is performed at the frame rate.

In a second aspect, a non-transitory computer readable storage medium has stored therein data representing instructions executable by a programmed processor for acquisition control for elasticity ultrasound imaging. The storage medium includes instructions for determining transducer, tissue, or transducer and tissue motion in response to activation of the elasticity ultrasound imaging, calculating a frame rate for the elasticity ultrasound imaging, calculating values for acquisition parameters based on the frame rate, and performing subsequent elasticity ultrasound imaging with the values.

In a third aspect, a system is provided for acquisition control for elasticity ultrasound imaging. A transmit beamformer and a receive beamformer are configured to generate pushing pulses and track displacements responsive to the pushing pulses. The transmit and receive beamformers are configured as a function of settings of acquisition parameters. A processor is configured to estimate motion different from the displacements responsive to the pushing pulses and to alter one or more of the settings as a function of a resolution based on the motion different from the displacements responsive to the pushing pulses. A display is operable to display an image, where the image is a function of the displacements and at least some of the displacements are responsive to the altered one or more settings.

The present invention is defined by the following claims, and nothing in this section should be taken as a limitation on those claims. Further aspects and advantages of the invention are discussed below in conjunction with the preferred embodiments and may be later claimed independently or in combination.

BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a flow chart diagram of one embodiment of a method for acquisition control for elasticity ultrasound imaging;
Figures 2 and 3 are examples of different numbers of pushing pulses used to control frame rate in elasticity imagine; and
Figure 4 is one embodiment of a system for acquisition control for elasticity ultrasound imaging.

## DETAILED DESCRIPTION OF THE DRAWINGS AND PRESENTLY PREFERRED EMBODIMENTS

Acquisition frame rate is automatically determined in acoustic radiation force impulse imaging, shear wave imaging, or other elasticity imaging. An appropriate acquisition time given detected motion is automatically determined. The acquisition parameters in the elasticity imaging are correspondingly adjusted to acquire the best images under the current motion conditions.

In one embodiment, transducer and/or tissue motion are determined while imaging. The system calculates an appropriate ARFI or shear wave frame rate for the amount of motion present, and acquisition parameters (e.g., line spacing, acoustic output, number of parallel receive channels or beams, number of push pulses, number of tracking pulses, cool down time, and/or number of B-mode reference frames) are automatically adjusted to achieve the desired frame rate. In another embodiment, the system automatically changes the ARFI or shear wave acquisition parameters to provide optimal temporal resolution, spatial resolution and/or image quality depending on the amount of motion present during acquisition.

The automatic setting of one or more acquisition parameters for elasticity imaging may be performed periodically or continuously while scanning, in real-time, for elasticity imaging. Alternatively, the automatic setting occurs once for single frame acquisition of an elasticity image. For example, the user triggers acquisition of a shear or ARFI image. The system detects the motion, sets the acquisition parameters based, at least in part, on the amount of motion, and then generates the elasticity image using the settings. The system may automatically adjust acquisition parameters in response to automatic triggering or upon request from the user (e.g., an "update" control).

Any motion detection may be used, such as B-mode, Doppler or elasticity-based motion detection. The desired frame rate may be determined algorithmically (e.g. Nyquist Sampling) or empirically.

When there is a lot of motion present, instead of increasing the frame rate in ARFI or shear wave imaging and compromising image quality, the system may automatically switch to another elasticity mode (e.g., ARFI to strain, shear to ARFI, shear to strain, etc.). When there is not a lot of motion present, instead of decreasing the frame rate in an elasticity mode, the system may automatically switch to another elasticity mode (e.g., strain to ARFI, strain to shear, ARFI to shear, etc.).

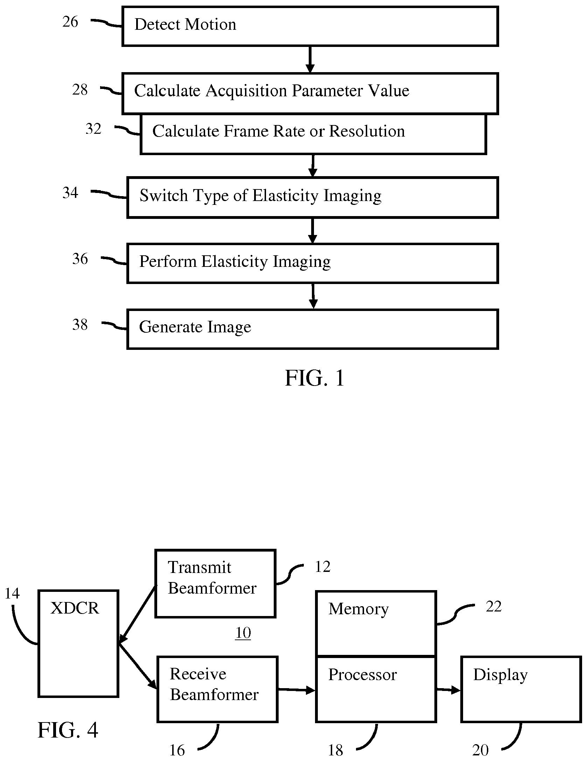

Figure 1 shows a method for acquisition control for elasticity ultrasound imaging. The amount of motion is detected prior to or during elasticity imaging. In response to the amount of motion, one or more acquisition parameters are set to achieve an appropriate frame rate and/or resolution (e.g., image quality). The setting of the acquisition parameters occurs automatically. Rather than relying on user guesses, one or more values are set by the system. Previously user set or default values may be altered for subsequent elasticity imaging by the system. The determination of motion and calculating of frame rate, resolution, and/or values of one or more acquisition parameters may occur without user entry of the values or alteration of already input values set to control the frame rate.

The method is implemented by the system of Figure 4 or a different system. For example, a processor, controller, or image processor of an ultrasound imaging system determines an amount of motion and calculates the frame rate, resolution and/or values of acquisition parameters. Transmit beamformers, memory, detectors and/or other devices may be used to acquire the data, perform one or more of the acts, and/or output the data. The processor may control the devices to perform the method of Figure 1.

Additional, different, or fewer acts may be provided. For example, the method is performed without generating an image in act 38. As another example, act 32 is not performed. The values of the acquisition parameters are mapped to the motion, effectively providing the elasticity imaging at a motion responsive frame rate but without calculating a specific frame rate to use. In another example, act 34 is not performed. In yet another example, acts for B-mode scanning or other types of scanning are provided, such as scanning to detect motion separately from performing the elasticity imaging.

The acts are performed in the order described or shown, but may be performed in other orders. Separate scans may be performed to detect the motion, so the order shown is used. Once the motion is determined and acquisition parameters calculated, then the elasticity imaging is performed. This order may be appropriate for either on-going or repeated performance of the elasticity scanning or for a one-shot approach where a single elasticity image is acquired in response to a user or automated trigger. In an alternative order, the elasticity imaging is performed. Data acquired as part of the elasticity imaging is used to detect motion. The calculated acquisition parameters are then used in subsequent elasticity scanning. This order may be appropriate for on-going or real-time elasticity scanning. The motion detection and calculation are repeated at any period.

In act 26, motion is detected. The motion is caused by movement of the patient relative to the transducer. Due to patient and/or transducer shift, the field of view also shifts. The plane or volume being imaged within the patient changes position within the patient because of the motion. For example, the sonographer may be searching for the region of interest, so moves the transducer around on the skin surface of the patient. Resulting images are viewed during the movement until the region of interest (e.g., organ) is located. Then, the amount of movement is reduced or movement ceases to generate images of the organ at the located region of interest.

Another source of motion is movement within the patient. For example, heart and/or lung movement causes organs or other tissue to shift position relative to other organs and/or other tissue. Internal tissue of the patient moves, such as the heart or heart walls moving cyclically. Greater temporal resolution (e.g., higher frame rate) is desired during transducer or tissue motion.

The motion is determined in response to activation of elasticity ultrasound imaging. For on-going elasticity imaging, the motion determination is repeated continuously or periodically, such a repeating for each or other integer number of elasticity images in a sequence. Alternatively, the motion determination occurs once for the on-going elasticity imaging. For one-shot elasticity imaging (i.e., acquire a single elasticity image in response to user activation), the motion is determined in response to user activation or other trigger of the elasticity scan. For example, a trigger button is depressed. Before elasticity imaging and in response to activation of the elasticity imaging, the motion is detected. Other triggers may be used, such as determining an amount of motion for altering acquisition parameters in response to detection of a sufficiently large motion. In one embodiment, any change in the elasticity imaging input by the user triggers motion determination.

The amount of motion is determined. The motion vector in one, two, or three-dimensions is calculated by a processor. The translation or lateral shift is found. In other embodiments, the amount of rotation and/or change in scale is determined. The magnitude of the motion indicates how much motion or change in position has occurred. Any period for determining the motion may be used, such as motion between sequential elasticity frames or images or motion between the same phases of different cycles in the heart cycle.

The motion is determined from any source. A sensor is used in one embodiment. For example, a magnetic position sensor on the transducer is used to detect motion of the transducer. In other embodiments, ultrasound data is used to detect the motion. Combinations of techniques for detecting motion may be used.

For detecting the motion from ultrasound data, B-mode data may be used. Two frames or images of B-mode data are relatively displaced by different amounts. For each displacement, a correlation of some or all of the data is calculated. The displacement (e.g., translation with or without rotation) with the greatest correlation provides the motion vector. In another embodiment, de-correlation indicates motion in an elevation direction. An amount of de-correlation between sequential frames or images without relative offset shows an amount of out-of-scan plane motion. In yet another embodiment, Doppler data is used. A global velocity is calculated, such as an average tissue velocity in an image or part of an image. The global velocity represents the amount of motion.

In one approach, the ultrasound data also used for elasticity imaging is used to detect the motion. Elasticity imaging uses a sequence of different acquisitions of data. For example, a reference frame of data is acquired (e.g., B-mode frame). A pushing pulse (e.g., ARFI pulse) is transmitted. For ARFI imaging, the same scan line with or without adjacent scan lines are then monitored with a sequence of acquisitions. For shear wave imaging, adjacent or laterally displaced scan lines are then monitored with a sequence of acquisitions. The sequences after the pushing pulse are used to detect localized displacement of tissue caused by the longitudinal or shear wave. The process may be repeated for other laterally spaced locations in a region of interest.

Due to the number of acquisitions performed for elasticity imaging, motion compensation may be used. A global motion or motion of tissue spaced away from the longitudinal or shear wave generated by the pushing pulse is monitored. This global motion or motion not due to the generated wave is subtracted from or accounted for in the calculations of localized motion used to detect elasticity. The same global motion or motion not due to the generated wave may be used as the detected motion for calculating acquisition parameters and/or frame rate.

In one embodiment of using the motion from motion correction in elasticity imaging, the motion is detected from correlation of data from the elasticity ultrasound imaging. One or an average reference frame of data is compared to a frame of data for tracking the wave displacement. To minimize distortion caused by the wave, the last frame or last few frames of the tracking after a given pushing pulse are used. The reference and tracking frames of data for elasticity imaging with respect to a same pushing pulse are used, but frames from different pushing pulses (e.g., reference frames from pushing pulses along the same scan line or adjacent scan lines) may be used. These last frames for tracking are likely to not include motion from the generated wavefront since the wavefront likely already passed. By finding the motion or offset with the greatest or sufficient correlation between the reference frame and the last tracking frame, the amount of motion is detected. By using all of the data, tissue data from one or more locations, or other sub-set, the global motion occurring between the reference frame and the tracking frame is detected.

The period between frames is known from the acquisition and includes sufficient time to transmit the pushing pulse and monitor or track local displacements due to the generated wavefront. The offset and the period may be used to calculate a velocity vector. Alternatively, the amount of motion is used without calculating a specific period and/or velocity. The regularity of the process may be used to normalize for time (i.e., since the time between reference frame and last tracking frame is the same for each repetition, the offset alone indicates the amount of motion relatively).

In another embodiment, the amount of de-correlation between on-going tracking frames is used. A sequence of tracking frames is acquired to find localized displacement caused by the shear or longitudinal wave. For tissue regions spaced from the likely locations of the wavefront or for all of the frames, an amount of de-correlation may indicate an amount of motion. The localized motion may cause some de-correlation, so a threshold may be used to identify sufficient de-correlation to indicate sufficient motion for changing acquisition parameters.

Motion as measured between any two times may be used. Alternatively, the motion is detected multiple times over time. For example, global displacement over time is detected using two or more detected motions (i.e., data from three or more times). A profile of the amount of global displacement over time is obtained. Any period may be used for the profile. The velocity, average, or other measure of motion (e.g., maximum of the profile) is calculated from the profile to provide the amount of motion. The magnitude of motion is determined from the profile.

Due to noise or other source of errors, a threshold may be applied in the detection of the motion. The threshold defines a range or a difference in a current amount of motion from a previous motion. If the motion changes sufficiently (e.g., less or more motion by a threshold amount), then the value or values of one or more acquisition parameters may be altered. If the motion changes by a little amount (e.g., less than the threshold amount), the previous value or values are still used for subsequent elasticity imaging. For an initial motion detection, the value or values are set based on the magnitude of that detected motion.

In act 28, one or more values are calculated for respective one or more acquisition parameters. A processor uses the amount of motion to determine a value for an acquisition parameter. The calculation is performed for a sufficient change in motion in an on-going process and/or for an initially detected motion for a one-shot or beginning of the on-going process. The amount of motion may be used to map directly to one or more values or collection of values of acquisition parameters. Alternatively, the amount of motion maps to a desired frame rate or resolution, which is then used to calculate the values of the acquisition parameters.

The value or values are initially set based on the motion. Alternatively, the user sets the values or defaults are used to set the value or values. The motion is used to alter or re-set the value or values. The value or values are altered to provide a greater or lesser frame rate than without the alteration.

The value or values are calculated to provide a desired frame rate and/or resolution based on the amount of motion. A higher frame rate is desired for greater motion so that the motion has less effect on the elasticity imaging and/or to better view the motion. The higher frame rate typically results in less spatial resolution and/or poorer image quality. Since there is a greater amount of motion, less quality and/or spatial resolution may be acceptable. Instead of setting frame rate based on the amount of motion, resolution may be set based on the amount of motion.

The calculation of the value or values uses an analytical calculation. For example, the Nyquist principle is used to determine a frame rate with sufficient sampling given a velocity. Other functions may be used. In other embodiments, the value or values are calculated using mapping or a look-up table. Different amounts of motion are mapped to different values or sets of values. An empirically established relationship of frame rate to motion may be found and used. For example, the frame rate desired by a sampling of users for each given amount or range of motions is found and used for mapping.

In one embodiment, the amount of motion is mapped directly to the value or values of the acquisition parameters. In other embodiments, the amount of motion is mapped to the desired frame rate and/or resolution in act 32. The value or values of the acquisition parameters are then selected to achieve the frame rate and/or resolution. For example, a frame rate for the elasticity imaging is calculated for a given amount of motion. A range of motions may map to a same frame rate, but different ranges of motions map to different frame rates in a step-wise relationship. Using a continuous or step-wise mapping, a greater frame rate is calculated for greater magnitude of motion, and a lesser frame rate is calculated for lesser magnitude.

Rather than or in addition to using frame rate, resolution or image quality may be used. The value or values to provide a desired resolution or quality given a particular amount of motion are calculated.

For controlling the frame rate, one or more values are set. A plurality of acquisition parameters defines the transmission, reception, and/or sequence for elasticity imaging. A single acquisition parameter may be used to adjust frame rate. More than one acquisition parameter may be used to adjust the frame rate, such as using two or more. In one embodiment, sets entirely defining the beamforming and sequence for the elasticity imaging are provided. Each set is associated with a different frame rate and/or motion. Between sets, one, more, or all of the values may be different. One or more of the values between sets may be the same. The motion is used to calculate the value or values by selecting one of the sets. Selecting one set as compared to another may result in just one or multiple values being altered for changing frame rate from one level to another. Alternatively, a value is altered without selecting an set.

Acquisition parameters are variables that affect the acquisition of the ultrasound data, such as the acquisition of data used for elasticity imaging. The acquisition parameters are for transmit operation, receive operation, or both. For example, the acquisition parameters are any beamformer setting. Any setting that controls an aspect of the acoustic energy transmitted from a transducer and/or any setting for forming a beam on receive may be used. The sequence used for elasticity imaging is responsive to the acquisition parameters, such as timing between transmissions and/or receptions being acquisition parameters. Once the system has determined the amount of motion present and/or an appropriate frame rate for that motion condition, then the processor calculates the elasticity sequence and corresponding values of the acquisition parameters to match the desired frame rate.

Some example acquisition parameters include a number of pushing pulses, a number of tracking scans responsive to each pushing pulse, a number of reference scans, a pulse repetition interval of the tracking scans, a pulse repetition interval of the reference scans, a duration of each pushing pulse, and a cool down time. These example acquisition parameters relate to frame rate, as represented by:

$$Frame_{pri} = M_{push} \times \left( N_{Track} \times Track_{pri} + L_{Ref} \times Ref_{pri} + Push_{time} \right) + Cool\_Down_{time}$$

The factors affecting the frame rate in ARFI imaging and shear imaging are included. $Frame_{pri}$ is the time required to capture a single frame of ARFI or shear data, $M_{push}$ is the number of push pulses in the frame, $N_{track}$ and $L_{ref}$ are the number of tracking and reference acquisitions, $Track_{pri}$ and $Ref_{pri}$ are their associated pulse repetition intervals, $Push_{time}$ is the time needed for a push pulse, and $Cool\_Down_{time}$ is any buffer time per frame to remain within acoustic output limits. Other acquisition parameters may affect the frame rate, such as the number of simultaneously transmitted tracking beams, the number of simultaneously received tracking beams, and/or the depth of the field of view.

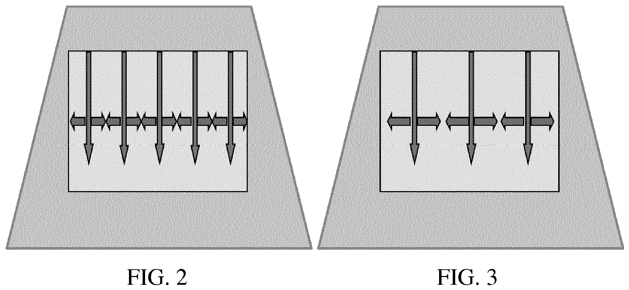

Acquisition or other imaging parameters are automatically traded to increase frame rate or reduce frame rate depending on the amount of motion present. The frame rate = $1/Frame_{pri}$ so anything that reduces $Frame_{pri}$ increases the frame rate. There are imaging compromises involved in any of these tradeoffs. Reducing the number of push pulses requires less dense spacing across a region of interest (compare Figure 2 with Figure 3 where the vertical arrows represent pushing pulses). Reducing the number of pushing pulses in a given region of interest may reduce the resolution in ARFI images and/or reduce the signal-to-noise ratio in shear wave images due to tracking the shear waves over longer distances. Reducing the number of tracking and/or reference pulses reduces the image quality in ARFI and shear wave images by reducing the accuracy of the displacement estimates. Reducing $Push_{time}$ (e.g., fewer cycles of the pushing pulse) reduces the image quality by reducing the amount of displacement in ARFI imaging or the amplitude of the shear wave generated in shear wave imaging. Reducing the cool down time necessitates reducing the amplitude of the push pulse. The reduction in amplitude reduces the amount of displacement in ARFI imaging or the amplitude of the shear wave generated in shear wave imaging.

For optimal image quality and resolution, a frame rate that is as low as possible in elasticity imaging is desired. Transducer motion or tissue motion may require the use of higher frame rates. By detecting the motion and using the detected motion to calculate the value or values for acquisition parameters, the optimal or acceptable tradeoff between frame rate and image quality and resolution is selected for the current imaging conditions. If there is a lot of transducer or tissue motion, the frame rate is increased as compared to the rate with little motion but the spatial resolution and image quality goes down. If the transducer is steady and/or there is little tissue motion, then the system provides higher resolution and higher quality elasticity images. The change in frame rate is relative to other frame rates previously used during a given or on-going elasticity imaging session. Alternatively, a frame rate is set based on motion for a given elasticity image. The frame rate relates to the amount of time over which the elasticity imaging acquisition occurs for a single frame or for multiple frames.

The number of pushing pulses, $M_{push}$, may have the most drastic effect on the frame rate. Figures 2 and 3 show examples of two different numbers of pushing pulses (5 in Figure 2 and 3 in Figure 3) for a given region of interest. The horizontal arrows represent shear waves generated by the pushing pulses. The shear waves are tracked over a limited distance due to a high push pulse density in the ROI of Figure 2 as compared to Figure 3. The frame rate in this example is 3 Hz. Where the system determines that a change should occur or a different number should be used initially, a different frame rate is used, such as 5 Hz. To achieve this frame rate, the number of push pulses is set to or reduced to three, as shown in Figure 3. As desired, the frame rate is at or changed to increase to 5 Hz, but the image quality would likely reduce due to the lower push pulse density in the region of interest and the need to track shear waves over longer distances (e.g., longer ranges for the horizontal arrows). A greater number of push pulses per region or image provides for a lower frame rate than a lesser number.

Other acquisition parameters may be used to control the frame rate instead of or with more refinement in combination with the number of pushing pulses. For example, the number of tracking scans responsive or performed after each pushing pulse is set. To detect elasticity, the displacements caused by the generated wave over time are determined. By using more tracking scans, greater temporal resolution may be provided for detecting elasticity. A greater number of tracking scans takes more time, reducing the frame rate. A lesser number of tracking scans takes less time, especially where detection of the return of tissue to a relaxed state is not tracked.

In act 34, another aspect of the acquisition sequence is calculated based on the motion. The type of elasticity imaging being used is set based on the amount of motion. Different types of elasticity imaging take different amounts of time to acquire a frame or image. For example, strain rate imaging may be performed at a B-mode frame rate (e.g., 15-30 Hz). ARFI imaging may require a greater lateral number of pushing pulses for a given region than shear wave imaging, but a single pushing pulse may be used to track the longitudinal wave over many depths in ARFI imaging as opposed to shear imaging repeating pushing pulses for different depths. Depending on the depth of the field of view, the frame rate for ARFI imaging may be more rapid than shear wave imaging.

For example, the user selects shear wave imaging. After activating the on-going or real time shear wave imaging, the user searches for the region of interest by moving the transducer. Due to the large amount of motion, the acquisition parameters are set to provide strain images instead of shear wave images. Once the region is found and the transducer is steadier, the processor detects less motion so switches the values of the acquisition parameters and type of elasticity imaging to the shear wave imaging. The values used for shear wave imaging may be altered over time due to motion as well.

In act 36, elasticity imaging is performed. ARFI imaging, shear imaging, strain imaging, or strain rate imaging is provided. The elasticity imaging is performed using the values of the acquisition parameters. One or more of the values are set or altered based, at least in part, on the detected motion, so the elasticity imaging is responsive to the motion. The frame rate and/or resolution of the elasticity imaging are based on the amount of motion.

The motion is detected, and the settings are calculated from the motion or altered based on the motion. Subsequent performance of the elasticity imaging uses the calculated settings. In one embodiment, the elasticity imaging is on-going or repeated during a same imaging session (e.g., for a same patient in a same 15 minute ultrasound examination). During the repetitions, the same or different settings are used. If the amount of motion changes sufficiently during the imaging, the settings are altered to change the frame rate during the imaging. The detection of the motion and calculating of the value or values are performed automatically and repetitively with the elasticity ultrasound imaging. The frame rate adjustment occurs as a background process using the processor or controller rather than the user having to change the values manually using a keyboard, sliders, knobs, or buttons. Merely moving the transducer may result in automatic change without further user input. Tissue motion, such as motion of the heart, may result in automatic change without further user input. The frame rate for acquiring a single or multiple frames of elasticity imaging data is set as an alteration of a current value based on an amount of change in motion or is set based on a mapping using the amount of motion without consideration for the current setting.

For performing the elasticity imaging act 36, an acoustic radiation force impulse (ARFI) beam is transmitted. An array of elements in an ultrasound transducer transmits the ARFI beam converted from electrical waveforms. The acoustic energy is transmitted to the tissue in a patient. The acoustic waveform is transmitted for generating a shear, longitudinal, or other wave as stress to displace tissue. The excitation is an ultrasound pushing pulse. The acoustic energy is focused to apply sufficient energy to cause generation of one or more waves travelling through the tissue from the focal locations. The acoustic waveform may itself displace the tissue. Other sources of stress may be used.

The shear wave or waves are generated at the focal region and propagate laterally, axially, and/or in other directions from the focal region. The waves may travel in multiple directions. The waves reduce in amplitude as the waves travel through the tissue.

To generate the wave, high amplitude or power excitations are desired. For example, the excitation has a mechanical index of close to but not exceeding 1.9 at any of the focal locations and/or in the field of view. To be conservative and account for probe variation, a mechanical index of 1.7 or other level may be used as the upper limit. Greater (e.g., MI exceeding 1.9) or lesser powers may be used.

The pushing pulse is transmitted with waveforms having any number of cycles. In one embodiment, one, most, or all of the waveforms for a transmit event have 100-2,000 cycles. The number of cycles is tens, hundreds, thousands, or more for the continuous transmit waveforms applied to the elements of the array for the pushing pulse. Unlike imaging pulses that are 1-5 cycles, the ARFI pushing pulse has a greater number of cycles to generate sufficient stress to cause the wave for displacing tissue with an amplitude sufficient to detect.

The length of the transmission in combination with the amplitude provides acoustic power to the tissue. This power may cause a rise in temperature in the tissue. Transmitting along the same or adjacent scan lines may cause the tissue to increase in temperature over time. Biological effects may include hyperthermia at tissue temperature of about 41-45°C, protein denaturation at temperatures above 43-45°C, and tissue necrosis at temperatures above 50°C. Tissue stiffness may be affected even at temperatures below 43-45°C. At temperatures above 43-45°C, increases in viscosity and/or stiffness may occur. At temperatures above 50°C, the tissue may have a high stiffness and/or high attenuation. Biological effects are limited by preventing a temperature increase of over 2 degrees Celsius. Alternatively, the transmissions may cause biological effects. By providing a cool down between pushing pulses, the temperature change may be limited.

The displacements caused by the pushing pulse are tracked. The tissue response is a function of the wave created by the ARFI beam and the tissue characteristics. The displacement of the tissue over time may be expressed as a convolution of the waveform and the tissue characteristics or response. The tissue response reflects viscoelastic properties of the tissue.

One or more viscoelastic properties are measured from displacement caused by the pushing pulse. In order to measure the viscoelastic properties, the displacement of the tissue over time in response to the pushing pulse is measured. The displacement of the tissue caused by the created wave or the ARFI pulse itself is determined over time. As the wave passes a given location, the tissue displaces by an amount or distance that increases to a peak amount and then decreases as the tissue returns to rest.

The tissue is scanned multiple times to determine the displacement, such as scanning a region at least three times to determine displacement at two different times. The tissue is scanned using any imaging modality capable of scanning for displacement during the tissue's response to the pushing waveform, such as during or after application of the ARFI pushing pulse. The scan occurs over a range of times where the desired waveform (e.g., shear wave) would be passing through the tissue.

For ultrasound scanning, the wave is detected at locations adjacent to and/or spaced from the focal region for the ARFI pushing pulse. To detect the displacement, ultrasound energy is transmitted to the tissue undergoing displacement, and reflections of the energy are received. To detect tissue response to waves in a region of interest, transmissions are made to the region, and detection is performed in the region. These other transmissions are for detecting the waves or displacement rather than causing the wave or displacement. The transmissions for detection may have lower power and/or short pulses (e.g., 1-5 carrier cycles) and use the same or different scan line as the ARFI beam. The transmissions for detection may have a wider beam profile along at least one dimension, such as laterally, for simultaneously forming receive samples along a plurality of scan lines. The wave or displacement may be monitored in one, two, or more directions.

A region of interest is monitored to detect the wave. The region of interest is any size. For example, the wave is detected along various depths of one or more lines in ARFI imaging. As another example, the displacements are tracked at each of a plurality of laterally spaced locations for a limited depth in shear wave imaging. By including a wider ARFI beam laterally, a greater number of scan lines may be monitored for axial displacement per ARFI beam. For example, eight or more (e.g., 16) scan lines are monitored for each ARFI pushing pulse.

The transmission and reception for detection or tracking are performed multiple times to determine change due to displacement over time. Any transmission and reception sequence may be used. The detection of displacement may be interleaved with other scanning, such as scanning different regions for displacement separately.

The displacement is calculated from the ultrasound scan data. The tissue moves between two scans. The data of one scan is translated in one, two, or three dimensions relative to the data in the other scan. For each possible relative position, an amount of similarity is calculated for data around a location. The amount of similarity is determined with correlation, such as a cross-correlation. A minimum sum of absolute differences or other function may be used. The spatial offset with the highest or sufficient correlation indicates the amount and direction of displacement for a given location. In other embodiments, a phase offset of data received from different times is calculated. The phase offset indicates the amount of displacement. In yet other embodiments, data representing a line (e.g., axial) at different times is correlated to determine a shift for each of a plurality of depths along the line.

Displacements are determined for a given location at different times, such associated with sequential scans. The displacement is determined with respect to an initial or reference frame of scan data (i.e., cumulative displacement). Alternatively, the displacement is determined from the immediately prior frame of scan data, such assigning the previous frame as the reference on an ongoing basis (i.e., incremental displacement). The temporal profile for a given location indicates displacement caused by the wave over time.

Where the focal region extends sufficiently for the desired measurements, a single ARFI pushing pulse is used. Where a broader region of interest exists, the pushing pulse and tracking may be repeated for different depths. The transmissions and receptions for displacement detection are interleaved with ARFI beams to scan different regions of tissue. To monitor a larger lateral region, pushing pulses and tracking are repeated for other locations. For each receive beam location, a time profile of motion information (i.e., displacements) is provided. A separate time profile is provided for each axial depth and/or lateral location.

The displacement information, with or without a time profile, is used to determine a characteristic of the tissue. The characteristic is determined at each location. Any characteristic may be determined, such as an elasticity, strain, shear velocity, longitudinal wave velocity, modulus, or other viscoelastic property. The displacements themselves may be used to represent the tissue, such as the magnitude of the peak displacement weighted by distance from the focal point of the pushing pulse.

In act 38, an image is generated. The image represents the tissue characteristic or property. The image is a function of the displacement. Using the localized displacements themselves or a characteristic derived from the displacements (e.g., shear modulus), information to be displayed is calculated. For example, a numerical or textual indication of the property may be displayed. In other embodiments, a plot and/or line fit and slope value are output. For example, displacement over time is displayed for each of one or more locations. The viscoelastic property is communicated to the user in the image. The image may be a graph, such as a plot of values as a function of location.

The image may additionally include a one, two, or three-dimensional representation of the property, displacement, or other wave information as a function of space or location. For example, the shear velocity throughout a region is displayed. Shear velocity values modulate color for pixels in a region in a gray-scale modulated B-mode image. The image may represent displacement information, such as shear or moduli (e.g., the shear moduli) for different locations. The display grid may be different from the scan grid and/or grid for which displacements are calculated. Color, brightness, luminance, hue, or other characteristic of pixels is modulated as a function of the information derived from the displacements.

In other embodiments, the displacements are used for shear wave velocity imaging or ARFI velocity imaging. The distribution of velocities in a two or three-dimensional region are determined and mapped to image values.

The frame rate for the elasticity images is based on the motion. Other imaging (e.g., B-mode or Doppler) may occur at other frame rates. By setting the acquisition parameters based on the amount of motion, the frame rate for the elasticity imaging is set. For on-going elasticity imaging or other repetition in elasticity imaging, the frame rate and/or resolution may change due to differences in the amount of motion. For single shot or a single given elasticity image, the frame rate (e.g., time to acquire) and/or resolution may be different depending on the amount of motion.

Figure 4 shows one embodiment of a system 10 for acquisition control for elasticity ultrasound imaging. Ultrasound generates tissue displacement, such as through creation of a shear or longitudinal wave, and scan data responsive to the tissue responding to the displacement is used to determine a property. The frame rate and/or resolution for the detection of the tissue property is controlled based on or as a function of an amount of detected motion.

The system 10 is a medical diagnostic ultrasound imaging system. In alternative embodiments, the system 10 is a personal computer, workstation, PACS station, or other arrangement at a same location or distributed over a network for real-time or post acquisition imaging.

The system 10 implements the method of Figure 1 or other methods. The system 10 includes a transmit beamformer 12, a transducer 14, a receive beamformer 16, an image processor 18, a display 20, and a memory 22. Additional, different or fewer components may be provided. For example, a user input is provided for manual or assisted designation of a region of interest for which information is to be obtained.

The transmit beamformer 12 is an ultrasound transmitter, memory, pulser, analog circuit, digital circuit, or combinations thereof. The transmit beamformer 12 is configured to generate waveforms for a plurality of channels with different or relative amplitudes, delays, and/or phasing. The waveforms are generated and applied to a transducer array with any timing or pulse repetition frequency. For example, the transmit beamformer 12 generates a sequence of pushing pulses for different laterally and/or range regions and a corresponding transmissions for tracking resulting displacements with ultrasound.

The transmit beamformer 12 connects with the transducer 14, such as through a transmit/receive switch. Upon transmission of acoustic waves from the transducer 14 in response to the generated waves, one or more beams are formed during a given transmit event. The beams are pushing pulses and/or tracking beams. For scanning tissue displacement, a sequence of transmit beams are generated to scan a one, two or three-dimensional region. Sector, Vector®, linear, or other scan formats may be used. The same region is scanned multiple times. The scanning by the transmit beamformer 12 occurs after transmission of the pushing pulse, but may include scanning for reference frames used in tracking before transmitting the pushing pulse. The same elements of the transducer 14 are used for both scanning and displacing tissue, but different elements, transducers, and/or beamformers may be used.

The transducer 14 is a 1-, 1.25-, 1.5-, 1.75- or 2-dimensional array of piezoelectric or capacitive membrane elements. The transducer 14 includes a plurality of elements for transducing between acoustic and electrical energies. For example, the transducer 14 is a one-dimensional PZT array with about 64-256 elements.

The transducer 14 connects with the transmit beamformer 12 for converting electrical waveforms into acoustic waveforms, and connects with the receive beamformer 16 for converting acoustic echoes into electrical signals. The transducer 14 transmits the pushing pulse and tracking beams. The waveforms are focused at a tissue region or location of interest in the patient. The acoustic waveforms are generated in response to applying the electrical waveforms to the transducer elements. For scanning with ultrasound to detect displacement, the transducer 14 transmits acoustic energy and receives echoes. The receive signals are generated in response to ultrasound energy (echoes) impinging on the elements of the transducer 14.

The receive beamformer 16 includes a plurality of channels with amplifiers, delays, and/or phase rotators, and one or more summers. Each channel connects with one or more transducer elements. The receive beamformer 16 applies relative delays, phases, and/or apodization to form one or more receive beams in response to each transmission for detection. Dynamic focusing on receive may be provided. The receive beamformer 16 outputs data representing spatial locations using the received acoustic signals. Relative delays and/or phasing and summation of signals from different elements provide beamformation. In alternative embodiments, the receive beamformer 16 is a processor for generating samples using Fourier or other transforms.

The receive beamformer 16 may include a filter, such as a filter for isolating information at a second harmonic or other frequency band relative to the transmit frequency band. Such information may more likely include desired tissue, contrast agent, and/or flow information. In another embodiment, the receive beamformer 16 includes a memory or buffer and a filter or adder. Two or more receive beams are combined to isolate information at a desired frequency band, such as a second harmonic, cubic fundamental, or other band.

The receive beamformer 16 outputs beam summed data representing spatial locations. Data for a single location, locations along a line, locations for an area, or locations for a volume are output. The data may be for different purposes. For example, different scans are performed for B-mode or tissue detection than for shear or longitudinal wave detection. Alternatively, the B-mode data is also used to determine displacement caused by a shear or longitudinal wave.

The processor 18 or a separate beamformer controller configures the beamformers 12, 16. By loading values into registers or a table used for operation, the values of acquisition parameters used by the beamformers 12, 16 for elasticity imaging are set. Any control structure or format may be used to establish the elasticity imaging sequence. The beamformers 12, 16 are caused to acquire data for elasticity imaging at a frame rate and/or with a resolution. Different values of one or more acquisition parameters may result in a different frame rate and/or resolution.

The processor 18 is a B-mode detector, Doppler detector, pulsed wave Doppler detector, correlation processor, Fourier transform processor, application specific integrated circuit, general processor, control processor, image processor, field programmable gate array, digital signal processor, analog circuit, digital circuit, combinations thereof or other now known or later developed device for detecting and processing information from beamformed ultrasound samples.

In one embodiment, the processor 18 includes one or more detectors and a separate processor. The separate processor is a control processor, general processor, digital signal processor, graphics processing unit, application specific integrated circuit, field programmable gate array, network, server, group of processors, data path, combinations thereof or other now known or later developed device for determining displacement and/or calculating tissue properties. The processor 18 is configured by software and/or hardware to perform the acts.

In one embodiment for elasticity imaging, the processor 18 estimates tissue displacement over time as a function of the output data from the receive beamformer 16. The displacements are estimated as a profile or data representing a curve of magnitude of displacement as a function of time. The displacement profile may be obtained by correlating or otherwise determining level of similarity between reference data and data obtained to represent the tissue at a different time.

The processor 18 is configured to calculate tissue characteristics form the displacements of the tissue over time. For example, a shear velocity is calculated from the displacement over time. The amount of displacement divided by the time provides velocity. In one embodiment, the processor 18 calculates viscosity and/or modulus. The processor 18 may calculate other properties, such as strain or elasticity. In yet other embodiments, the processor 18 determines the maximum displacement or other characteristic of displacement or the displacement profile as the characteristic.

The processor 18 is also configured to estimate motion. The motion estimated is different from the localized displacements responsive to the pushing pulses or wavefronts created by the pushing pulses. The motion estimated is a global motion or motion difference from the wavefront for elasticity imaging. Motion of tissue spaced from the locations of wavefront monitoring and/or motion normalized to account for the displacements by the wavefront (e.g., motion detected from the tissue when not under the influence of any or much of the wavefront) is estimated.

The processor 18 or a separate processor is configured to set acquisition parameters of the beamformers 12, 16 for elasticity imaging. The values of the parameters are calculated by look-up, calculated from scratch using an equation or function, or calculated as an amount of alteration from a current value. The values are calculated as a function of the estimated motion. One or more of the settings are altered or initially set as a function of a resolution or frame rate. The resolution or frame rate desired is based on the motion. For example, a lower resolution is to be provided in the image with the settings based on a greater amount of the motion. Alternatively, the amount of motion may be mapped directly to the values, where the values result in a desired frame rate and/or resolution.

The processor 18 generates and outputs image or display values mapped from the property to the display 20. For example, the shear modulus or other value is determined. A text or numerical indication of the property is displayed to the user. A graph of the property over time may be displayed.

In one embodiment, the property is displayed as a function of location. Values, graphs, and/or tissue representations may be displayed using the displacements at different locations. For a representation of the tissue, the magnitude of the tissue characteristic modulates the color, hue, brightness, and/or other display characteristic for different pixels representing a tissue region. The processor 18 determines a pixel value (e.g., RGB) or a scalar value converted to a pixel value. The image is generated as the scalar or pixel values. The image may be output to a video processor, look-up table, color map, or directly to the display 20.

The display 20 is a CRT, LCD, monitor, plasma, projector, printer or other device for displaying an image or sequence of images. Any now known or later developed display 20 may be used. The display 20 is operable to display one image or a sequence of images. The display 20 displays two-dimensional images or three-dimensional representations. The display 20 displays one or more images representing the tissue characteristic or other information derived from displacements. As an example, a two-dimensional image or three-dimensional representation of displacement or tissue characteristics as a function of location is displayed. Alternatively or additionally, the image is a graph, a number, or text representation of a value or graph. For example, a shear modulus, strain, elasticity or other value or graph is displayed as the image.

The rate of display of a sequence of elasticity images is the same as or based on the acquisition frame rate. Where the frame rate changes, the rate of display of images also changes. For a given region of interest, the elasticity images are displayed at any frame rate, such as 3-6 Hz. The frame rate used in a given situation is based on the detected motion and set using the values of the acquisition parameters.

Similarly, the spatial resolution and/or image quality is based, in part, on the acquisition parameters. The spatial resolution and/or quality of the elasticity image may be based on the detected motion and set using the values of the acquisition parameters. Different acquisition parameters may result in different spatial resolutions, temporal resolution, or image quality for the displayed image. A change in acquisition parameters of a sequence may result in images of the sequence having different spatial resolution, frame rate, and/or image quality. Where the values of the acquisition parameters are altered, the resulting detected displacements for elasticity imaging are responsive to the alteration.

The processor 18, the receive beamformer 16, and the transmit beamformer 12 operate pursuant to instructions stored in the memory 22 or another memory. The instructions configure the system for performance of the acts of Figure 1. The instructions configure the processor 18, the receive beamformer 16, and/or the transmit beamformer 12 for operation by being loaded into a controller, by causing loading of a table of values (e.g., elasticity imaging sequence), and/or by being executed. The transmit beamformer 12 is configured by the instructions to cause generation of a pushing beam and tracking beams. The receive beamformer 16 is configured by the instruction to acquire data for tracking. The processor 18 is configured to detect an amount of motion and set the values of the acquisition parameters for elasticity imaging based, at least in part, on the amount of motion. The processor 18 is also configured to measure tissue displacement or characteristic and generate an elasticity image.

The memory 22 is a non-transitory computer readable storage media. The instructions for implementing the processes, methods and/or techniques discussed herein are provided on the computer-readable storage media or memories, such as a cache, buffer, RAM, removable media, hard drive or other computer readable storage media. Computer readable storage media include various types of volatile and nonvolatile storage media. The functions, acts, or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing, and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU or system.

**Claims**

1. A method for acquisition control for elasticity ultrasound imaging, the method comprising:

   detecting (26) motion of an ultrasound transducer, a patient being scanned by the ultrasound transducer, or both;
   calculating (28), with a processor (18), a value of an acquisition parameter, the value calculated to provide a frame rate based on an amount of the detected motion; and
   performing (36) elasticity ultrasound imaging using the value of the acquisition parameter, the elasticity ultrasound imaging being performed at the frame rate.

2. The method of claim 1 wherein detecting (26) the motion comprises detecting (26) motion from correlation of data from the elasticity ultrasound imaging.

3. The method of claim 2 wherein detecting (26) the motion comprises detecting (26) from a global motion vector between a reference frame acquired before generation of a longitudinal or shear wave in the patient and a last tracking frame acquired after generation of the longitudinal or shear wave.

4. The method of claim 1 wherein detecting (26) the motion comprises detecting (26) the motion as part of motion compensation of the elasticity ultrasound imaging.

5. The method of claim 1 wherein detecting (26) the motion comprises detecting (26) a profile of an amount of displacement over time.

6. The method of claim 1 wherein calculating (28) the value comprises selecting (32) a frame rate based on the motion and calculating (28) the value for the selected frame rate,

or

wherein calculating (28) the value comprises calculating (28) a number of pushing pulses for a region, a greater number providing a lower frame rate than a lesser number,

or

wherein calculating (28) the value comprises calculating (28) a number of tracking scans responsive to each pushing pulse, a greater number providing a lower frame rate than a lesser number.

7. The method of claim 1 wherein calculating (28) the value comprises calculating (28) the value and additional values, the value and the additional values comprising at least three from the group of: a number of pushing pulses, a number of tracking scans responsive to each pushing pulse, a number of reference scans, a pulse repetition interval of the tracking scans, a pulse repetition interval of the reference scans, a duration of each pushing pulse, and a cool down time.

8. The method of claim 1 wherein detecting (26) the motion comprises detecting (26) the motion with the amount above a threshold, and wherein calculating (28) comprises altering the value to provide a greater frame rate than without the altering

or

wherein detecting (26) the motion comprises detecting (26) the motion with the amount below a threshold, and wherein calculating (28) comprises altering the value to provide a lesser frame rate than without the altering.

9. The method of claim 1 wherein performing (36) the elasticity ultrasound imaging comprises performing (36) repetitively and wherein detecting (26) the motion and calculating (28) the value are performed automatically and repetitively with the elasticity ultrasound imaging.

10. The method of claim 1 wherein performing (36) the elasticity ultrasound imaging comprises performing (36) shear wave imaging or performing (36) acoustic radiation force imaging.

11. The method of claim 1 wherein calculating (28) the value comprises calculating (28) the value to provide a resolution based on the amount of the detected motion.

12. The method of claim 1 further comprising switching (34) from a first type of elasticity imaging to a second type of elasticity imaging based on the amount of the motion.

13. In a non-transitory computer readable storage medium having stored therein data representing instructions executable by a programmed processor (18) for acquisition control for elasticity ultrasound imaging, the storage medium comprising instructions for:

determining (26) transducer, tissue, or transducer and tissue motion in response to activation of the elasticity ultrasound imaging;
calculating (32) a frame rate for the elasticity ultrasound imaging based on an amount of the determined motion;
calculating (28) values for acquisition parameters based on the frame rate; and
performing (36) subsequent elasticity ultrasound imaging with the values.

14. The non-transitory computer readable storage medium of claim 13 wherein the determining (26) and both calculatings (32, 28) are performed automatically without user input of the values.

15. The non-transitory computer readable storage medium of claim 13 wherein determining (26) the motion comprises determining a magnitude of the motion, and wherein calculating (32) the frame rate comprises calculating a greater frame rate for greater magnitude and a lesser frame rate for lesser magnitude.

16. A system for acquisition control for elasticity ultrasound imaging, the system comprising:

a transmit beamformer (12) and a receive beamformer(16) configured to generate pushing pulses and track displacements responsive to the pushing pulses, the transmit and receive beamformers (12, 16) configured as a function of settings of acquisition parameters;
a processor (18) configured to estimate motion different than the displacements responsive to the pushing pulses, and to alter one or more of the settings as a function of a resolution based on the motion different than the displacements responsive to the pushing pulses; and
a display (20) operable to display an image, the image being a function of the displacements, at least some of the displacements responsive to the altered one or more settings.

**17.** The system of claim 16 wherein the processor (18) is configured to provide a lower resolution in the image with the settings based on a greater amount of the motion.

**Patentansprüche**

**1.** Verfahren zur Erfassungssteuerung für Elastizitäts-Ultraschall-Bildgebung, wobei das Verfahren umfasst:

Erfassen (26) von Bewegung eines Ultraschallwandlers, eines Patienten, der durch den Ultraschallwandler gescannt wird, oder beidem;
Berechnen (28), mit einem Prozessor (18), eines Wertes eines Erfassungsparameters, wobei der Wert berechnet wird, um eine Bildrate zu liefern, die auf einem Betrag der erkannten Bewegung basiert; und
Durchführen (36) von Elastizitäts-Ultraschall-Bildgebung unter Verwendung des Wertes des Erfassungsparameters, wobei die Elastizitäts-Ultraschall-Bildgebung bei der Bildrate durchgeführt wird.

**2.** Verfahren nach Anspruch 1, wobei das Erfassen (26) der Bewegung das Erfassen (26) von Bewegung aus Korrelation von Daten aus der Elastizitäts-Ultraschall-Bildgebung umfasst.

**3.** Verfahren nach Anspruch 2, wobei das Erfassen (26) der Bewegung das Erfassen (26) aus einem globalen Bewegungsvektor zwischen einem Bezugsbild, das vor Erzeugung einer Longitudinal- oder Scherwelle in dem Patienten erfasst wurde, und einem letzten Verfolgungsbild umfasst, das nach Erzeugung der Longitudinal- oder Scherwelle erfasst wurde.

**4.** Verfahren nach Anspruch 1, wobei das Erfassen (26) der Bewegung das Erfassen (26) der Bewegung als Teil von Bewegungskompensation der Elastizitäts-Ultraschall-Bildgebung umfasst.

**5.** Verfahren nach Anspruch 1, wobei das Erfassen (26) der Bewegung das Erfassen (26) eines Profils eines Betrags einer Verschiebung über der Zeit umfasst.

**6.** Verfahren nach Anspruch 1, wobei das Berechnen (28) des Wertes das Auswählen (32) einer Bildrate, die auf der Bewegung basiert, und das Berechnen (28) des Wertes für die ausgewählte Bildrate umfasst,

oder
wobei das Berechnen (28) des Wertes das Berechnen (28) einer Anzahl von Drückimpulsen für einen Bereich umfasst, wobei eine höhere Anzahl eine niedrigere Bildrate liefert als eine geringere Anzahl,
oder
wobei das Berechnen (28) des Wertes das Berechnen (28) einer Anzahl von Verfolgungs-Scans umfasst, die auf jeden Druckimpulse reagieren, wobei eine höhere Anzahl eine niedrigere Bildrate liefert als eine geringere Anzahl.

**7.** Verfahren nach Anspruch 1, wobei das Berechnen (28) des Wertes das Berechnen (28) des Wertes und zusätzlicher Werte umfasst, wobei der Wert und die zusätzlichen Werte wenigstens drei umfassen aus der Gruppe aus: einer Anzahl von Drückimpulsen, einer Anzahl von Verfolgungs-Scans, die auf jeden Drückimpulse reagieren, einer Anzahl von Bezugs-Scans, einem Pulswiederholintervall der Verfolgungs-Scans, einem Pulswiederholintervall der Bezugs-Scans, einer Dauer jedes Drückimpulses, und einer Abkühlzeit.

**8.** Verfahren nach Anspruch 1, wobei das Erfassen (26) der Bewegung das Erfassen (26) der Bewegung mit dem Betrag oberhalb eines Schwellenwerts umfasst, und wobei das Berechnen (28) das Verändern des Wertes umfasst, um eine höhere Bildrate als ohne dem Verändern zu liefern,

oder

wobei das Erfassen (26) der Bewegung das Erfassen (26) der Bewegung mit dem Betrag unterhalb eines Schwellenwerts umfasst, und wobei das Berechnen (28) das Verändern des Wertes umfasst, um eine geringere Bildrate als ohne dem Verändern zu liefern.

9. Verfahren nach Anspruch 1, wobei das Durchführen (36) der Elastizitäts-Ultraschall-Bildgebung das wiederholte Durchführen (36) umfasst und wobei das Erfassen (26) der Bewegung und Berechnen (28) des Wertes automatisch und wiederholt mit der Elastizitäts-Ultraschall-Bildgebung durchgeführt werden.

10. Verfahren nach Anspruch 1, wobei das Durchführen (36) der Elastizitäts-Ultraschall-Bildgebung das Durchführen (36) von Scherwellenbildgebung oder Durchführen (36) von Schallstrahlungskraftbildgebung umfasst.

11. Verfahren nach Anspruch 1, wobei das Berechnen (28) des Wertes das Berechnen (28) des Wertes umfasst, um eine Auflösung zu liefern, die auf dem Betrag der erkannten Bewegung basiert.

12. Verfahren nach Anspruch 1, ferner umfassend das Umschalten (34) von einer ersten Art von Elastizitätsbildgebung auf eine zweite Art von Elastizitätsbildgebung basierend auf dem Betrag der Bewegung.

13. In einem nicht-flüchtigen computerlesbaren Speichermedium, in dem Daten gespeichert sind, die Anweisungen wiedergeben, die durch einen Prozessor (18) ausführbar sind, der zur Erfassungssteuerung für Elastizitäts-Ultraschall-Bildgebung programmiert ist, wobei das Speichermedium Anweisungen umfasst zum:

Erfassen (26) von Schallwandler-, Gewebe-, oder Schallwandler- und Gewebebewegung in Reaktion auf Aktivierung der Elastizitäts-Ultraschall-Bildgebung;
Berechnen (32) einer Bildrate für die Elastizitäts-Ultraschall-Bildgebung, die auf einem Betrag der bestimmten Bewegung basiert;
Berechnen (28) von Werten für Erfassungsparameter, die auf der Bildrate basieren; und
Durchführen (36) von anschließender Elastizitäts-Ultraschall-Bildgebung mit den Werten.

14. Nicht-flüchtiges computerlesbares Speichermedium nach Anspruch 13, wobei das Erfassen (26) und beide Berechnungen (32, 28) automatisch ohne Benutzereingabe der Werte durchgeführt wird.

15. Nicht-flüchtiges computerlesbares Speichermedium nach Anspruch 13, wobei das Erfassen (26) der Bewegung das Erfassen einer Größe der Bewegung umfasst, und wobei das Berechnen (32) der Bildrate das Berechnen einer höheren Bildrate für höhere Größe und einer geringeren Bildrate für geringere Größe umfasst.

16. System zur Erfassungssteuerung für Elastizitäts-Ultraschall-Bildgebung, wobei das System umfasst:

einen Sende-Strahlformer (12) und einen Empfangs-Strahlformer (16), die dazu eingerichtet sind, Drückimpulse zu erzeugen und Verlagerungen zu verfolgen, die auf die Drückimpulse reagieren, wobei die Sende- und Empfangs-Strahlformer (12, 16) als eine Funktion von Einstellungen von Erfassungsparametern eingerichtet sind;
einen Prozessor (18), der dazu eingerichtet ist, Bewegung zu schätzen, die von den Verlagerungen, die auf die Drückimpulse reagieren, verschieden ist, und eine oder mehrere der Einstellungen als eine Funktion von einer Auflösung zu verändern, die auf der Bewegung basiert, die von den Verlagerungen, die auf die Drückimpulse reagieren, verschieden ist; und
eine Anzeige (20), die dazu betrieben werden kann, ein Bild anzuzeigen, wobei das Bild eine Funktion der Verlagerungen ist, wobei wenigstens einige der Verlagerungen auf die veränderten eine oder mehrere Einstellungen reagieren.

17. System nach Anspruch 16, wobei der Prozessor (18) dazu eingerichtet ist, eine niedrigere Auflösung in dem Bild mit den Einstellungen zu liefern, die auf einem höheren Betrag der Bewegung basieren.

**Revendications**

1. Procédé de commande d'acquisition pour une élastographie ultrasonore, le procédé comprenant :

la détection (26) du mouvement d'un transducteur ultrasonique, d'un patient en cours de balayage par le transducteur ultrasonore, ou de l'un et l'autre ;

le calcul (28), avec un processeur (18), d'une valeur d'un paramètre d'acquisition, la valeur étant calculée pour fournir une fréquence d'images sur la base d'une quantité du mouvement détecté ; et

l'exécution (36) d'une élastographie ultrasonore en utilisant la valeur du paramètre d'acquisition, l'élastographie ultrasonore étant exécutée à la fréquence d'images.

2. Procédé selon la revendication 1, dans lequel la détection (26) du mouvement comprend la détection (26) du mouvement à partir d'une corrélation de données provenant de l'élastographie ultrasonore.

3. Procédé selon la revendication 2, dans lequel la détection (26) du mouvement comprend la détection (26) à partir d'un vecteur de mouvement global entre une image de référence acquise avant génération d'une onde longitudinale ou de cisaillement chez le patient et une dernière image de suivi acquise après génération de l'onde longitudinale ou de cisaillement.

4. Procédé selon la revendication 1, dans lequel la détection (26) du mouvement comprend la détection (26) du mouvement dans le cadre d'une compensation de mouvement de l'élastographie ultrasonore.

5. Procédé selon la revendication 1, dans lequel la détection (26) du mouvement comprend la détection (26) d'un profil d'une quantité de déplacement au fil du temps.

6. Procédé selon la revendication 1, dans lequel le calcul (28) de la valeur comprend la sélection (32) d'une fréquence d'images sur la base du mouvement et le calcul (28) de la valeur pour la fréquence d'images sélectionnée,

ou

dans lequel le calcul (28) de la valeur comprend le calcul (28) d'un nombre d'impulsions de poussée pour une région, un nombre plus grand fournissant une fréquence d'images plus basse qu'un nombre plus petit,

ou

dans lequel le calcul (28) de la valeur comprend le calcul (28) d'un nombre de balayages de suivi qui réagissent à chaque impulsion de poussée, un nombre plus grand fournissant une fréquence d'images plus basse qu'un nombre plus petit.

7. Procédé selon la revendication 1, dans lequel le calcul (28) de la valeur comprend le calcul (28) de la valeur et de valeurs supplémentaires, la valeur et les valeurs supplémentaires comprenant au moins trois parmi le groupe de : un nombre d'impulsions de poussée, un nombre de balayages de suivi qui réagissent à chaque impulsion de poussée, un nombre de balayages de référence, un intervalle de répétition d'impulsion des balayages de suivi, un intervalle de répétition d'impulsion des balayages de référence, une durée de chaque impulsion de poussée et un temps de refroidissement.

8. Procédé selon la revendication 1, dans lequel la détection (26) du mouvement comprend la détection (26) du mouvement avec la quantité supérieure à un seuil, et dans lequel le calcul (28) comprend la modification de la valeur pour fournir une fréquence d'images plus grande que sans la modification

ou

dans lequel la détection (26) du mouvement comprend la détection (26) du mouvement avec la quantité inférieure à un seuil, et dans lequel le calcul (28) comprend la modification de la valeur pour fournir une fréquence d'images plus petite que sans la modification

9. Procédé selon la revendication 1, dans lequel l'exécution (36) de l'élastographie ultrasonore comprend l'exécution (36) de façon répétitive et dans lequel la détection (26) du mouvement et le calcul (28) de la valeur sont exécutés automatiquement et de façon répétitive avec l'élastographie ultrasonore.

10. Procédé selon la revendication 1, dans lequel l'exécution (36) de l'élastographie ultrasonore comprend l'exécution (36) d'une imagerie d'onde de cisaillement ou l'exécution (36) d'une imagerie de force de rayonnement acoustique.

11. Procédé selon la revendication 1, dans lequel le calcul (28) de la valeur comprend le calcul (28) de la valeur pour fournir une résolution basée sur la quantité du mouvement détecté.

12. Procédé selon la revendication 1, comprenant en outre la commutation (34) d'un premier type d'élastographie à un second type d'élastographie sur la base de la quantité du mouvement.

**13.** Support de stockage non transitoire lisible par ordinateur dans lequel sont stockées des données représentant des instructions exécutables par un processeur (18) programmé pour la commande d'acquisition pour l'élastographie ultrasonore, le support de stockage comprenant des instructions pour :

déterminer (26) un mouvement d'un transducteur, d'un tissu, ou d'un transducteur et d'un tissu en réponse à l'activation de l'élastographie ultrasonore ;
calculer (32) une fréquence d'images pour l'élastographie ultrasonore sur la base de la quantité du mouvement déterminé ;
calculer (28) des valeurs pour des paramètres d'acquisition sur la base de la fréquence d'images ; et
exécuter (36) une élastographie ultrasonore ultérieure avec les valeurs.

**14.** Support de stockage non transitoire lisible par ordinateur selon la revendication 13, dans lequel la détermination (26) et l'un et l'autre des calculs (32, 28) sont exécutés automatiquement sans saisie des valeurs par l'utilisateur.

**15.** Support de stockage non transitoire lisible par ordinateur selon la revendication 13, dans lequel la détermination (26) du mouvement comprend la détermination d'une amplitude du mouvement, et dans lequel le calcul (32) de la fréquence d'images comprend le calcul d'une fréquence d'images plus grande pour une amplitude plus grande et une fréquence d'images plus petite pour une amplitude plus petite.

**16.** Système de commande d'acquisition pour une élastographie ultrasonore, le système comprenant :

un formeur de faisceaux de transmission (12) et un formeur de faisceaux de réception (16) configurés pour produire des impulsions de poussée et des déplacements de suivi en réponse aux impulsions de poussée, les formeurs de faisceaux de transmission et de réception (12, 16) configurés en fonction de réglages de paramètres d'acquisition ;
un processeur (18) configuré pour estimer un mouvement différent des déplacements en réponse aux impulsions de poussée, et pour modifier un ou plusieurs des réglages en fonction d'une résolution sur la base du mouvement différent des déplacements en réponse aux impulsions de poussée ; et
un affichage (20) exploitable pour afficher une image, l'image étant une fonction des déplacements, au moins certains des déplacements réagissant au(x) réglage(s) modifié(s).

**17.** Système selon la revendication 16, dans lequel le processeur (18) est configuré pour fournir une résolution plus basse dans l'image avec les réglages sur la base d'une quantité de mouvement plus grande.

26 — | Detect Motion |

28 — | Calculate Acquisition Parameter Value |
32 — | Calculate Frame Rate or Resolution |

34 — | Switch Type of Elasticity Imaging |

36 — | Perform Elasticity Imaging |

38 — | Generate Image |

FIG. 1

14 — XDCR

Transmit Beamformer — 12

10

Receive Beamformer — 16

Memory — 22

Processor — 18

Display — 20

FIG. 4

FIG. 2

FIG. 3

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1782736 A1